# EUROPEAN PATENT APPLICATION

(11) **EP 3 944 858 A1**
(43) Date of publication of application: **02.02.2022**
(21) Application number: 20188648.8
(22) Date of filing: 30.07.2020
(51) Int. Cl.: A61K 31/445, A61K 31/7016, A61P 3/00, A61P 25/28

(54) **COMBINATION PRODUCT CONTAINING TREHALOSE AND MIGLUSTAT FOR TREATING LYSOSOMAL DISEASES**

(71) Applicant: Beyond Batten Disease Foundation, Austin TX 78763 (US)
(72) Inventor: KERKOVICH, Danielle, AUSTIN, TX Texas 78763 (US)
(74) Representative: Regimbeau

(57) **Abstract**

The present invention is based on the finding that parenteral administration of Trehalose leads to surprisingly high amounts of Trehalose that reach and accumulate in the brain compared to other routes, where it inhibits neuronal cell death and reduces neuroinflammation in a mouse model of a lysosomal disease (CLN3). It is also based on the finding that the trehalase (the enzyme responsible for the metabolism of trehalose) inhibitor Miglustat has a more pronounced beneficial effect on neuronal cell death, on its own, when orally administered at low and high doses in this mouse model and that the effect is even more pronounced in the presence of Trehalose. The present invention therefore relates to a combination product containing parenteral Trehalose and oral Miglustat, for separate administration, for treating lysosomal diseases such as the CLN3 disease. In particular, the invention concerns a combination product comprising Trehalose and Miglustat, or any pharmaceutically acceptable salt thereof, for simultaneous, separated, or staggered use for treating a lysosomal storage disorder or a disorder characterized by lysosomal dysfunction in a subject in need thereof, wherein Trehalose is administered parenterally and Miglustat is administered orally.

## Description

### SUMMARY OF THE INVENTION

The present invention is based on the finding that parenteral administration of Trehalose leads to surprisingly high amounts of Trehalose that reach and accumulate in the brain compared to other routes, where it inhibits neuronal cell death and reduces neuroinflammation in a mouse model of a lysosomal disease (CLN3). It is also based on the finding that the trehalase (the enzyme responsible for the metabolism of trehalose) inhibitor Miglustat has a more pronounced beneficial effect on neuronal cell death, on its own, when orally administered at low and high doses in this mouse model and that the effect is even more pronounced in the presence of Trehalose. The present invention therefore relates to a combination product containing parenteral Trehalose and oral Miglustat, for separate administration, for treating lysosomal diseases such as the CLN3 disease.

### BACKGROUND OF THE INVENTION

Lysosomes are membrane-bound cell organelles central to degradation processes in animal cells. Extracellular materials such as microorganisms taken up by phagocytosis, macromolecules by endocytosis, and unwanted cell organelles, fuse with lysosomes and are broken down to their basic molecules. Thus, lysosomes are the recycling units of a cell. Lysosomes are also responsible for cellular homeostasis for their role in secretion, plasma membrane repair, cell signaling, and energy metabolism.

The essential role of lysosomes in cellular degradation processes puts these organelles at the crossroads of several cellular processes, with significant implications for health and disease. Defects in one of 60 lysosomal enzymes, transmembrane proteins or other components of this organelle, prevent the breakdown of target molecules, and are responsible for more than 60 different human genetic diseases, which are collectively known as lysosomal storage disorders. The large number and variety of human pathological conditions that are characterized, if not caused by aberrant lysosomal functions, underscores the critical importance of the autophagy-lysosome pathways to cellular metabolism. In these diseases as well as diseases characterized by lysosomal dysfunction, undegraded materials accumulate within the lysosomes, contributing to the presence or severity of disease ranging from lysosomal storage disorders to neurodegenerative diseases, to cancer, to cardiovascular disease.

For instance, the neuronal ceroid lipofuscinoses (NCLs), lysosomal storage disorders also known as Batten disease, are a group of neurodegenerative disorders considered the most common of the neurogenetic storage diseases, with a prevalence of 1 in 12,500 in some populations. There are currently no cures or approved treatments for any of the 14 forms of Batten disease.

CLN3 disease is an ultra-rare, genetic, lysosomal storage disease that primarily affects the nervous system and is fatal. Children with CLN3 disease develop normally, even excelling in school until ages 4-7 years, when progressive vision loss becomes noticeable (Aberg et al., 2011; Bozorg et al., 2009). Concomitantly, or shortly thereafter, parents report personality changes and behavioral issues. Typically, within 2-3 years after symptom onset, total vision loss occurs and seizures begin. This is followed by declining speech, progressive loss of motor coordination and cardiac involvement. Psychosis, hallucinations and/or dementia can appear anytime during the disease. Eventually, children become wheelchair-bound, then bedridden and die in their late teens or early twenties (Ostergaard, 2016).

The pathological hallmark of CLN3 disease is the accumulation of incompletely digested material which causes an autophagic block leading to progressive axonal degeneration and neuron loss which is amplified by chronic neuroinflammation (Nixon and Yang, 2012). Evidence from animal models and cell culture from both animal and CLN3 patient cells demonstrates that accumulation of pathologic material, neuron loss (apoptosis) and neuroinflammation are inhibited by the clearance of protein and lipid aggregates (Palmieri et al., 2017a; Palmieri et al., 2017b; Settembre et al., 2011). Additionally, recent preclinical evidence suggests that these defects in autophagy may underlie the enhanced levels of α-synuclein oligomers, gangliosides GM1, GM2, and GM3 as well as reduced levels of sphingomyelin and autophagy observed in cellular models of CLN3 disease (Kang et al., 2014).

A key cellular homeostatic pathway implicated in CLN3 disease (Cao et al., 2006; Radke et al., 2018) and a myriad of other lysosomal storage disorders is autophagy. Autophagy is vital for the maintenance of energy and tissue homeostasis by degrading damaged or excess intracellular components such as aggregation-prone proteins, lipids, and organelles, and recycling the breakdown products. The nervous system appears to be particularly susceptible to the effects of defective lysosomal autophagy, likely due to a combination of the long-lived nature of post-mitotic neurons placing particular stress on protein-clearing processes, the extreme polarization of many neurons, and the high metabolic requirements of neurons leading to higher levels of oxidative damage in lysosomes via the Fenton reaction. Autophagy deficiency in neurons and the subsequent failure to prevent accumulation of abnormal proteins leads to neurodegeneration (Nikoletopoulou et al., 2015), which is a likely mechanism underlying the neuropathology observed in CLN3 disease.

While the primary cellular function of the CLN3 protein is still not clear, CLN3 deficiency has been linked to defects in the maturation and fusion of autophagosomes and endolysosomal compartments, lysosomal pH, and the motility of late endosomes and lysosomes, most likely through interactions with Hook1 and microtubule motor protein complexes (Fossale et al., 2004; Luiro et al., 2004; Cao et al., 2006; Uusi-Rauva et al., 2008; Uusi-Rauva et al., 2012), proliferation and apoptosis (Cotman and Staropoli, 2012; Cárcel-Trullols et al., 2015).

The Batten disease, and lysosomal storage diseases in general, are known to involve degeneration of neuronal brain cells. It is therefore of primary importance that their proposed treatment can pass the blood-brain barrier and effectively reach the brain, where it should remain at an effective concentration for a sufficient time to achieve a significant therapeutic effect.

It has been previously proposed to treat patients suffering from lysosomal diseases such as the San Filippo disease with oral uptake of Trehalose (Thesis of V.Mauri et al., 2014, WO 2017/185010, Palmieri et al). Yet, in some of these studies, Trehalose was shown not to reach the brain, when orally administered (Mauri's thesis). According to Mauri's thesis, this could be due to the fact that Trehalose is hydrolyzed by the intrinsic enzyme Trehalase at the epithelial brush border in the small intestine what causes only a small fraction of any orally administered dose to reach blood stream or tissues (see also in Cendret et al.; Tanaka et al., 2004).

Other studies have disclosed that Trehalose has limited access to the brain, as its passage would be blocked by the blood-brain barrier (Lee et al, 2018). To counteract this, some authors have proposed to use exceedingly high and continuous oral intake of Trehalose, in order to achieve the desired concentration in the brain tissue (Mauri's thesis, WO2017/136922). However, high dose of Trehalose was shown to induce cytotoxic effects (Khalifeh et al., 2018). Also, it was proposed to combine Trehalose with a Trehalase inhibitor such as Miglustat (WO2017/185010), or to administer Trehalose directly in the brain (Mauri's thesis).

Although *in vitro* models have been useful to demonstrate a possible mode of action for Trehalose's efficacy *in vivo*, given Trehalose's typical post-ingestion digestive and metabolic fate, *in vivo* data are needed to demonstrate intact Trehalose absorption, distribution, metabolism and elimination after its administration and to identify adequate and clinically relevant routes of administration. The present invention solved this need. As a matter of fact, the present inventors have studied the concentration of circulating Trehalose and assessed which location (brain or periphery) it can reach effectively, depending on its route of administration. By doing so, they demonstrated that, contrary to what was suggested in the prior art, even when parenterally (and not only intracerebrally) administered, Trehalose can be found in relatively high levels in the brain, where it has an effect on neuronal cell death, neuroinflammation and microglial activation. More importantly, they show that the brain level of Trehalose is higher when Trehalose is parenterally administered, as compared with an oral administration. This surprising finding enables to consider using this new route of administration for treating lysosomal diseases, instead of the usual oral intake. This will allow to lower the dose of injected Trehalose, thereby avoiding any side effect that could be attributed to the ingestion of high doses of Trehalose (Khalifeh et al., 2018).

In addition, the authors demonstrated that oral miglustat is able to increase the concentration of parenterally-administered trehalose in the blood, the liver and in the brain, hence reinforcing the ADME properties of Trehalose.

Based on these surprising results, the inventors now propose a novel and optimized therapeutic regimen using parenteral administration of Trehalose, said regimen providing a higher therapeutic efficacy in the treatment of disorders involving abnormal lysosomal storage, and CLN3 in particular.

### DETAILED DESCRIPTION OF THE INVENTION

Trehalose, a known pharmacological inducer of autophagy and lysosomal pathways, increases the life span and normalizes behavioral and neuropathological aggregates in animal models of Parkinson's disease (Khalifeh et al., 2019), Huntington's disease (Sarkar and Rubinsztein, 2008), and Alzheimer disease (Du et al., 2013; Tien et al., 2016; Portbury et al., 2017). The mechanisms for Trehalose-induced autophagy induction in CLN3 were previously unknown until BBDF-sponsored studies in a mouse model of CLN3 disease and in cells from patients and mice with CLN3 disease. These studies demonstrated that Trehalose enhances the clearance of proteolipid aggregates *via* modulation of transcription factor EB (TFEB), a master regulator of lysosomal pathways, governing lysosomal biogenesis and metabolism (Sardiello et al., 2009; Palmieri et al., 2017a; Palmieri et al., 2017b), autophagy (Settembre et al., 2011), lysosomal exocytosis (Medina et al., 2011) and proteostasis (Song et al., 2013).

Trehalose may further enhance autophagy through inhibition of the SLCA2 family of glucose receptors, resulting in activation of an AMPK-dependent autophagy pathway (Dehay et al., 2010; Uchida et al., 2014; DeBosch et al., 2016). By inducing autophagy, Trehalose protects cells against pro-apoptotic insults (Sarkar et al., 2007) and may act as a chemical chaperone to prevent protein misfolding contributing to aggregate formation (Perucho et al., 2012; Sarkar et al., 2014; Zhang et al., 2014).

The present inventors have shown that, contrary to what was suggested in the prior art, parenterally administered Trehalose can be found in relatively high levels in brain, where it has an effect on neuronal cell death, neuroinflammation and microglial activation. More importantly, they show that the brain level of Trehalose is higher when Trehalose is parenterally administered, as compared with an oral administration. This surprising finding enables to consider using this new route of administration for treating lysosomal diseases, instead of the usual oral intake.

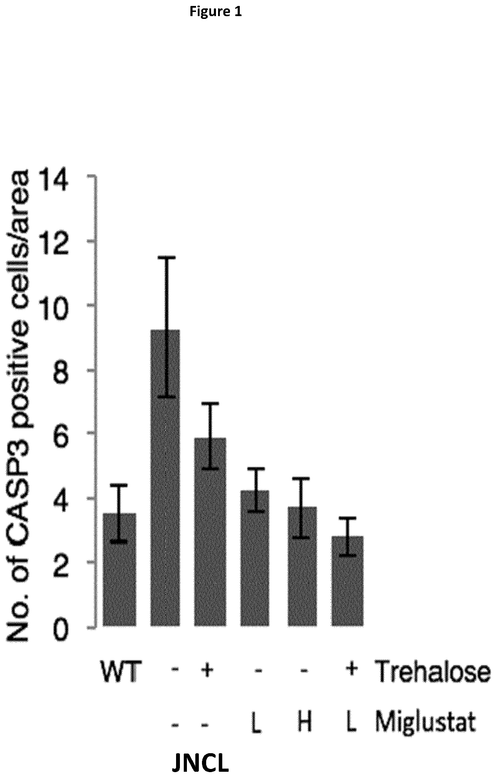

The present inventors have moreover investigated the effect of the Trehalase inhibitor Miglustat *in vivo* in a mouse model of Batten disease. They have shown that, surprisingly, Miglustat has also a strong effect on its own on neuronal cell death, neuroinflammation and microglial activation, even at low dose (Figures 1-3). They also show that the level of Trehalose in the brain is much higher when Miglustat and Trehalose are separately administered to a subject. This could be due to a stabilizing effect of Miglustat on Trehalose, in the brain, or an inhibition of trehalase, the enzyme degrading Trehalose.

Thus, the combination of Trehalose and Miglustat, through the activation of autophagy by Trehalose, the ability of Miglustat to reduce both ganglioside accumulation and inflammation, and the potential increased exposure of Trehalose *via* trehalase inhibition or direct stabilization (by Miglustat) suggest that the combination of Trehalose and Miglustat has the potential to slow the progression of CLN3 disease.

The present inventors propose a novel and optimized therapeutic regimen using parenteral administration of Trehalose and oral administration of Miglustat. This particular regimen is likely to display a higher therapeutic efficacy in the treatment of disorders involving abnormal lysosomal storage, and CLN3 in particular, because Trehalose accumulates in the brain of mice that are treated with this combination product. This is all the more surprising as it was thought that Trehalose is hydrolyzed by the intrinsic enzyme trehalase at the epithelial brush border in the small intestine (Cendret et al.; Tanaka et al., 2004; Thesis of Mauri et al.), what causes only a small fraction of any enterally administered dose to reach blood stream, neuronal, brain or muscle tissues.

In a first aspect, the present invention relates to a combination product comprising Trehalose and Miglustat, or any pharmaceutically acceptable salt thereof, for simultaneous, separated, or staggered use for treating a lysosomal storage disorder or a disorder characterized by lysosomal dysfunction in a subject in need thereof, wherein Trehalose is administered parenterally and Miglustat is administered orally.

The present invention also discloses a method for treating or alleviating a lysosomal storage disorder, or a disorder characterized by lysosomal dysfunction, or at least one symptom associated therewith, in a human subject in need thereof, said method comprising parenterally administering to said subject a therapeutically effective amount of Trehalose or a pharmaceutical formulation comprising a therapeutically effective amount of Trehalose, and orally administering a therapeutically amount of Miglustat.

The term "Trehalose" as used herein refers to the form of the Trehalose compound *per se*, as well as any other form such as a salt, polymorph, enantiomer, stereoisomer, conformer, solvate, ester, amide, prodrug, analog, derivative, or the like, provided that said salt, polymorph, ester, enantiomer, stereoisomer, conformer, solvate, amide, prodrug, analog, or derivative is suitable pharmacologically of a trehalose analog capable of inhibiting the AKT enzyme. Any crystalline form of Trehalose is herewith encompassed, notably those disclosed in US20010033888. The crystal structure of the rhomboid Trehalose dihydrate has been published by Taga et al. in 1972. Anhydrous form of Trehalose can also be used in the composition of the invention. In the composition product of the invention, the Trehalose can be a Trehalose analog. The Trehalose analog can be selected from lentzTrehalose A, lentzTrehalose B, and lentzTrehalose C (Wada et al).

Trehalose, also known as mycose or tremalose, is a stable, non-reducing disaccharide with two glucose molecules linked in a 1,1 configuration. The structure of Trehalose is diagrammed below (Table 1). Trehalose has protein-stabilizing properties, and is extensively used in many applications as a stabilizer of frozen food, in freeze-drying of biological systems and cells, as a stabilizer of therapeutic parenteral proteins, and as an excipient in tablets and IV solutions. Trehalose is recognized as a GRAS (Generally Regarded as Safe) food ingredient by the FDA and is listed on the USP-NF (United States Pharmacopoeia National Formulary), EP (European Pharmacopoeia) and JP (Japanese Pharmacopoeia). The safety and toxicity of Trehalose has been extensively investigated, and the substance was found to be safe when administered both orally and intravenously, in doses that are substantially higher than the intended therapeutic dose.

Preferably, Trehalose is substantially free of contaminants resulting from isolation and purification process of Trehalose. Trehalose may be isolated by extraction from dry yeast or the like; by enzymatic production and isolation; and by the culturing of microorganisms. As such, Trehalose is preferably substantially free of such contaminants as enzymes, organic solvents such as ammonium, acetonitrile, acetamide, alcohol (e.g., methanol, ethanol, or isopropanol), TFA, ether, or other contaminants used in a process for preparing and purifying trehalose. The term "substantially" free of contaminants may refer to Trehalose having a contaminant content of preferably less than 0.5%, less than 0.3%, less than 0.25%, less than 0.1%, less than 0.05%, less than 0.04%, less than 0.03%, less than 0.02%, less than 0.01%, less than 0.005%, less than 0.003%, or less than 0.001% of the total weight of the Trehalose. Methods of determining the content of contaminants is known in the art and may be determined by conventional methods such as gas chromatography. Preferably, the residual solvents in the purified Trehalose of the invention are less than the limits set in the ICH guidelines. For example, the purified trehalose contains <5000 ppm ethanol (e.g., <140 ppm), and/or <3000 ppm methanol.

In a preferred embodiment, the Trehalose used in the combination product of the invention is anhydrous or hydrated, for example mono or dihydrated. In a most preferred embodiment, the Trehalose used in the combination product of the invention is Trehalose dihydrate.

Miglustat, on the other hand, is a well-known member of the family of N-alkylated imino sugars. It has been approved in several countries, including in EU and in the US (Butters et al, 2007). Synthesis of this molecule is explained for example in EP1896083. Only one polymorphic crystalline form was observed. This crystalline anhydrous form has been characterized by various analytical techniques like FTIR, XRPD (X-Ray Powder Diffraction), DSC (Differential Scanning Calorimeter) and TGA (Thermo Gravimetric Analysis) during the manufacture and at release.

The term "Miglustat" as used herein, therefore refers to the compound N-butyl-deoxynojirimycin (N-butyl DNJ), also named 1,5-(butylimino)-1,5-dideoxy-D-glucitol, N-butyl-deoxynojirimycin or (2R,3R,4R,5S)-1-butyl-2-(hydroxymethyl)piperidine-3,4,5-triol, as well as any other form such as a salt, polymorph, enantiomer, stereoisomer, conformer, solvate, ester, amide, prodrug, analog, derivative, or the like, provided said salt, enantiomer, stereoisomer, conformer, solvate ester, amide, prodrug, analog, or derivative is capable of inhibiting the Trehalase enzyme and/or act as a chaperone protein for Trehalose.

The characteristics of the two components of the combination product of the invention are more preferably those summarized on Table I:

| | |
|---|---|
| **Structural formula** | Trehalose dihydrate |
| | 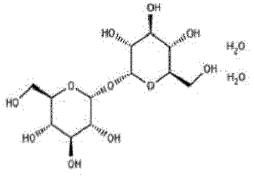 |
| | Miglustat |
| | 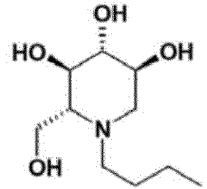 |
| **Chemical name** | Trehalose: α,α-trehalose dihydrate; α-D-glucopyranosyl α-D-glucopyranoside dihydrate |
| | Miglustat: (2R,3R,4R,5S)-1-butyl-2-(hydroxymethyl) piperidine-3,4,5-triol |
| **Molecular formula** | Trehalose : C₁₂H₂₂O₁₁·2H₂0 |
| | Miglustat: C₁₀H₂₁NO₄ |
| **Molecular weight** | Trehalose: 378.33 |
| | Miglustat: 219.281 |
| **CAS Reference** | Trehalose: 6138-23-4 |
| | Miglustat: 72599-27-0 |
| **Physiochemical description** | Trehalose: White, odorless, non-hygroscopic crystalline powder |
| | Miglustat: White crystalline powder |
| **Solubility** | Trehalose: 68.9 g/100 g H₂O at 20°C |
| | Miglustat: Highly soluble in water (>1000 mg/mL as a free base). |

The term "lysosomal storage disorders and disorders characterized by lysosomal dysfunction" may be used herein to describe any condition that may be caused by impaired lysosomal metabolism or any condition which exhibits or is exacerbated by lysosomal dysfunction. There are at least 60 known lysosomal storage disorders and many other disorders characterized by lysosomal dysfunction which may affect different parts of the body, including the skeleton, brain, skin, heart, and central nervous system. Additional disorders characterized by lysosomal dysfunction continue to be identified. Some of them are highlighted on Table II below.

| **Disease** | **Deficiency** |
|---|---|
| ***Primary lysosomal hydrolase defect*** | |
| Gaucher disease | Glucocerebrosidase |
| GM1 gangliosidosis | GM1-β-galactosidase |
| Tay-Sachs disease | β-Hexosaminidase A |
| Sandhoff disease | β-Hexosaminidase A+B |
| Fabry disease | α-Galactosidase A |
| Krabbe disease | β-Galactosyl ceramidase |
| Niemann-Pick disease Types A and B | Sphingomyelinase |
| Metachrοrnatic leukodystrophy | Arylsulpliatase A |
| MPS IH (Hurler syndrome) | α-Iduronidase |
| MPS IS (Scheie syndrome) | α-Idurondase |
| MPS II (Hunter syndrome) | Iduronate sulphatase |
| MPS IIIA (Sanfilippo A syndrome) | Heparan sulphamidase |
| MPS IIIB (Sanfilippo B syndrome) | N-Acetylglucosaminidase |
| MPS IIIC (Sanfilippo C syndrome) | Acetyl CoA:α-glucosaminide *N*-acetyltransferase |
| MPS IIID (Sanfilippo D syndrome) | *N*-acetyl glucosamine-6-sulphatase |
| MPS IV A (Morquio A disease) | Acetyl galactosamine-6-sulphatase |
| MPS IVB (Morquio B disease) | β-Galactosidase |
| MPS V (redesignated MPS IS) | |
| MPS VI (Maroteaux Lamy Syndrome) | Acetyl galactosamine-4-sulphatase (ARSB) |
| MPS VII (Sly Syndrome) | β-Glucuronidase |
| MPS IX | Hyaluronidase |
| Farber disease | Acid ceramidase |
| Cholesteryl ester storage disease | Acid lipase |
| Pompe disease (type II) | α1,4-glucosidase |
| Aspartylglucosaminuria | Glycosylasparaginase |
| Fucosidosis | α-Fucosidase |
| α-Mannosidosis | α-Mannosidase |
| β-Mannosidosis | β-Mannosidase |
| Schindler disease | *N*-acetylgalactosaminidase |
| Sialidosis | α-Neuraminidase |
| Infantile neuronal ceroid lipofuscinoses (CLN1) | Palmitoyl protein thioesterase |
| Late infantile neuronal ceroid lipofuscinosis (CLN2) | Carboxypeptidase |

| ***Post-translational processing defect in lysosomal enzymes*** | |
|---|---|
| Mucosulphatidosis (MSD) | Multiple sulphatases |

| ***Trafficking defect in lysosomal enzymes*** | |
|---|---|
| Mucolipidosis type II (I-cell disease) | *N*-acetyl glucosamine phosphoryl transferase |
| Mucolipidosis type IIIA (pseudo-Hurler polydystrophy) | *N*-acetyl glucosamine phosphoryl transferase |
| Mucolipidosis type IIIG | |
| ***Defect in lysosomal enzyme protection*** | |
| Galactosialidosis | Protective protein cathepsin A (PPCA) (β-galactosidase and neuraminidase) |

| ***Defect in soluble non-enzymatic lysosomal proteins*** | |
|---|---|
| Niemann-Pick type C | NPC2 |
| GM2 activator protein deficiency, Variant AB | GM2 activator protein |
| Sphingolipid activator protein (SAP) deficiency | Sphingolipid activator protein |
| Neuronal ceroid lipofuscinosis (CLN5) | |

| ***Transmembrane (non-enzyme) protein defect*** | |
|---|---|
| Danon disease | Lysosome-associated membrane protein 2 (LAMP2) |
| Niemann-Pick Type C | NPC1 |
| Cystinosis | Cystinosin |
| Infantile free sialic acid storage disease (ISSD) | Sialin |
| Salla disease (free sialic acid storage) | Sialin |
| Juvenile neuronal ceroid lipofuscinosis (CLN3, Batten disease) | |
| Neuronal ceroid lipofuscinoses (CLN6 and CLN8) | |
| Mucolipidosis type IV | Mucolipin |

| ***Unclassified*** | |
|---|---|
| Neuronal ceroid lipofuscinoses (CLN4 and CLN7) | |

Non-limiting examples of lysosomal storage disorders and disorders characterized by lysosomal dysfunction that may be treated using combination product and methods of the present disclosure include : Juvenile Neuronal Ceroid Lipofuscinosis (JNCL, juvenile Batten or CLN3 disease), Aspartylglucosaminuria, Cystinosis, Fabry Disease, San Filippo disease, Gaucher Disease Types I, II, and III, Glycogen Storage Disease II (Pompe Disease), GM2-Gangliosidosis Type I (Tay Sachs Disease), GM2-Gangliosidosis Type II (Sandhoff Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN11 disease, CLN12 disease, CLN13 disease, and CLN14 disease.

The mucopolysaccharide storage disease is preferably selected from : Hurler syndrome (MPS IH), Hurler-Scheie syndrome (MPS IH/S), Scheie syndrome (MPS IS; Mucopolysaccharidosis type V), Hunter syndrome (MPS II), Sanfilippo syndrome A (MPS IIIA), Sanfilippo syndrome C (MPS IIIC), Sanfilippo syndrome D (MPS IIID), Morquio Type A, Morquio Type B, Maroteaux-Lamy (MPS VI), Sly diseases (MPS VII), and Natowicz syndrome (MPS IX).

In a preferred embodiment, the combination product of the invention enables to treat Juvenile Neuronal Ceroid Lipofuscinosis (JNCL, juvenile Batten or CLN3 disease), Aspartylglucosaminuria, Cystinosis, San Filippo disease, Glycogen Storage Disease II (Pompe Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN12 disease, CLN13 disease, and CLN14 disease.

In a preferred embodiment, the combination product of the invention enables to treat Neuronal Ceroid Lipofuscinosis (CLN disease), in particular CLN1 disease, CLN2 disease, CLN3 disease, CLN4 disease, CLN5 disease, CLN6 disease, CLN7 disease, CLN8 disease, CLN10 disease, CLN11 disease, CLN12 disease, CLN13 disease, or CLN14 disease.

In a most preferred embodiment, the combination product of the invention enables to treat Juvenile Neuronal Ceroid Lipofuscinosis (JNCL, or Batten disease, or CLN3).

JNCL is the most prevalent neurodegenerative disorder of childhood. A hallmark of JNCL is the intralysosomal accumulation of ceroid lipopigments in most nerve cells and in various extra-cerebral tissues, indicating impairment of autophagy-lysosome pathways. JNCL presents with vision failure and hearing loss, and progresses to include seizures, motor dysfunction, and dementia. JNCL patients experience relentless physical and cognitive decline that leads to death by the third decade of life. As such, treating JNCL using the combination product of the present invention will prevent intralysosomal accumulation of ceroid lipopigments in nerve cells and in various cerebral and extra-cerebral tissues of a subject having JNCL, or will reduce or eliminate intralysosomal accumulation of the ceroid lipopigments. Methods of determining intralysosomal accumulation of ceroid lipopigments are known in the art and may be as described in the examples. Additionally, treating JNCL using the combination product of the invention will prevent, reverse, or arrest cognitive decline in a subject. Methods of determining cognitive decline resulting from JNCL in a subject are known in the art. For instance, treating JNCL using the combination product of the invention may prevent, reverse, or arrest vision failure. Treating JNCL using the combination product of the invention may also prevent, reverse, or arrest hearing loss. Treating JNCL using the combination product of the invention may also reduce the severity and/or intensity of seizures. Additionally, treating JNCL using the combination product of the invention may improve or prevent motor dysfunction. Treating JNCL using the combination product of the invention may also improve or prevent dementia.

Treating JNCL using the combination product of the invention closure may also extend the lifespan of a subject in need thereof. Using the combination product of the invention, the median life span of a subject having JNCL may be extended by about 1%, 5%, 10%, 15%, 20%, 30%, 40%, 50%, 60%, 70%, 80%, or about 90% or to the point where the disorder no longer is a factor in longevity of the subject. For instance, the combination product of the invention may extend the median lifespan of a subject with JNCL by about 60%, 65%, 70%, 75%, 80%, 85%, or about 90% or to the point where the disorder no longer is a factor in longevity of the subject. Alternatively, the combination product of the invention may extend the median lifespan of a subject with JNCL by about 20%, 25%, 30%, 35%, 40%, 45% or about 50%. The combination product of the invention may also extend the median lifespan of a subject with JNCL by about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or about 25%.

The term "parenterally" as herein defined refers to a route of administration where the desired effect is systemic and the active agent (herein defined as Trehalose), is administered by routes other than the digestive tract, for example intravenous, subcutaneous, intradermal, intramuscular and intraperitoneal administration. In a preferred embodiment, the composition containing Trehalose is for intravenous or sub-cutaneous administration.

In the combination product of the invention, Trehalose is contained in a composition that is suitable for parenteral administration. Such compositions are known in the art. This composition contains, apart from Trehalose, at least one pharmaceutically acceptable additive, carrier, excipient or diluent.

In a preferred embodiment, Trehalose is the single active principle in the composition for parenteral administration that is contained in the combination product of the invention.

In a preferred embodiment, the composition of the invention comprising Trehalose is formulated as an injectable intravenous dosage form for intravenous administration. It can be, for example, formulated as a dry powder that has to be diluted extemporaneously in the appropriate pharmaceutically acceptable solution.

In a more preferred embodiment, the dosage form is an infusion fluid comprising Trehalose. Trehalose infusion fluid formulations are known in the art. A Trehalose infusion fluid can typically comprise from about 1 to about 1000 mg/mL Trehalose, from about 10 to about 500 mg/mL Trehalose, or from about 50 to about 150 mg/mL Trehalose. In some aspects, a Trehalose infusion fluid according to the invention can comprise from about 80 to about 100 mg/mL Trehalose. In some embodiments, the concentration of Trehalose in the formulation in the combination product of the invention is between about 0.1 % (w/v) to about 50 % (w/v), preferably between 5 % (w/v) to about 15 % (w/v), in particular of about 8-10 % (w/v). In some embodiments, the injectable composition comprising Trehalose has an osmolality of from about 280 to about 330 mOsm/Kg.

When administered intravenously, the Trehalose can be administered in said subject at a dosage ranging from about 0.25 g/kg to about 1 g/kg, preferably from about 0.25 g/kg to about 0,75 g/kg. At such doses, Trehalose is preferably administered once or twice weekly, more preferably once weekly. In other embodiments, an intravenous Trehalose composition is administered at a dose ranging from about 0.1 g/kg to about 1 g/Kg or from about 0.2 g/kg to about 0.8 g/Kg.

When administered intravenously, a composition comprising Trehalose may be administered over a period of about 25, 50, 60, 70, 75, 80, 85, 90, 95 to about 120 minutes or 180 minutes (three hours). More preferably, when administered intravenously, a composition comprising Trehalose may be administered within less than 90 minutes, preferably for about 25 minutes to about three hours, more preferably for about 40 minutes to about 90 minutes, most preferably for about 50 minutes to about 70 minutes.

The injectable composition of the invention, comprising Trehalose or a Trehalose analog, preferably comprises a low level of endotoxins. Bacterial endotoxins are lipopolysaccharides (LPS), components of Gram-negative bacterial cell walls known to cause fevers and disease when injected into the bloodstream. Bacterial endotoxins are heat stable and toxicity is not dependent on the presence of the bacterial cell. Since many therapeutics, including Trehalose, may be made in bacteria, endotoxin testing is employed to ensure a therapeutic product is endotoxin-free. A composition comprising Trehalose may contain less than 1.0, 0.9, 0.8, 0.75, 0.7, 0.6, 0.5, 0.4, 0.3, 0.2, 0.1 or less endotoxin units per mL. Preferably, a composition comprising Trehalose contains less than 0.75 endotoxin units per mL of solution.

Further, a composition comprising Trehalose is preferably administered intravenously so that the maximum endotoxin level is less than 5 EU per kilogram of body weight per hour. In particular, a composition comprising Trehalose may be administered intravenously such that the endotoxin level is less than about 1, 2, 3, or less than about 4 endotoxin units per kilogram of body weight per hour.

In one particular embodiment, the Trehalose of the combination product of the invention is administered once weekly by an intravenous infusion extending for about 50 minutes to about 70 minutes. In one most preferred embodiment, the Trehalose is administered once weekly at a dosage ranging from about 0.25 g/Kg to about 0.75 g/kg, by an intravenous infusion extending for about 50 to about 70 minutes.

On another hand, in the context of the invention, the Miglustat can be administered to the subjects in need thereof by any oral formulations, such as in food, beverage, capsules, tablets, syrup, etc. Oral compositions generally may include an inert diluent or an edible carrier. Oral compositions may be enclosed in gelatin capsules or compressed into tablets. For the purpose of oral therapeutic administration, the active compound may be incorporated with excipients and used in the form of tablets, troches, or capsules. Oral compositions may also be prepared using a fluid carrier for use as a mouthwash, wherein the compound in the fluid carrier is applied orally and swished and expectorated or swallowed. Pharmaceutically compatible binding agents and/or adjuvant materials may be included as part of the composition. The tablets, pills, capsules, troches, and the like, may contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. For administration by inhalation, the compounds are delivered in the form of an aerosol spray from a pressured container or dispenser which contains a suitable propellant, e.g., a gas such as carbon dioxide, or a nebulizer.

Preferably, in the combination product of the invention, Miglustat is administered in a capsule. Capsules of Miglustat are commercialized by Actelion Pharmaceuticals under the name Zavesca^{®}. The nonclinical and clinical safety of this product in patients is well-established. In addition, Zavesca is approved in the EU for safe use in adults and children with Niemann-Pick Type C disease, a related neurodegenerative lysosomal storage disease at doses up to 200 mg TID.

In the combination product of the invention, Miglustat can be administered at a dosage ranging from about 75 mg per day to about 600 mg per day. In a preferred embodiment, Miglustat is administered at a dosage ranging from about 75mg per day to 600 mg per day.

When Trehalose is administered in combination with Miglustat, the oral dosage form for Miglustat is preferably one or more capsule(s), each comprising from about 10 to about 500 mg Miglustat, or from about 50 to about 300 mg Miglustat. In some aspects, a Miglustat capsule that can be used in the combination product of the invention can comprise 100, 150, 200, 250 or 300mg of Miglustat.

In an even more preferred embodiment, Miglustat in the combination product of the invention is administered three times per day (t.i.d.) to six times per day with three to six capsules containing each between 90 and 110 mg of Miglustat, preferably 100 mg of Miglustat.

More precisely, the Miglustat of the combination product of the invention can be orally administered :
- at a dosage ranging from about 175 mg once a day to about 300 mg t.i.d if the subject is twelve years of age or older or if the subject is less than twelve years of age and has a body surface area (BSA) of > 1.25 m²,
- at a dosage ranging from about 175 mg once a day to about 300 mg twice a day if the subject is less than twelve years of age and has a BSA of > 0.88-1.25 m²,
- at a dosage ranging from about 75 mg once a day to about 150 mg t.i.d. if the subject is less than twelve years of age and has a BSA of > 0.73-0.88 m²,
- at a dosage ranging from about 75 mg once a day to about 150 mg twice a day if the subject is less than twelve years of age and has a BSA of > 0.47-0.73 m²,
- or at a dosage ranging from about 75 mg to about 150 mg once a day if the subject is less than twelve years of age and has a BSA of > 0.47 m².

In some specific embodiments of the combination product of the invention, Trehalose is intravenously administered once weekly at a dosage ranging from about 0.25 g/Kg to about 0.75 g/Kg by an intravenous infusion extending for about 50 to about 70 minutes and the Miglustat is orally administered at a dosage ranging from about 100 mg t.i.d to about 100 mg six times per day, or at any lower dosage described above, depending on the age, and body surface area of the subject in need of the treatment.

In a particular embodiment, the present disclosure provides a method of treating juvenile Neuronal Ceroid Lipofuscinosis (Batten Disease or CLN3) in a subject in need thereof, the method comprising administering Trehalose intravenously once weekly at a dosage ranging from 0.25 g/Kg to about 0.75 g/Kg by an intravenous infusion extending for about 50 to about 70 minutes and orally administering Miglustat at a dosage ranging from about 175 mg once a day to about 300 mg t.i.d if the subject is twelve years of age or older or if the subject less than twelve years of age and has a body surface area (BSA) of > 1.25 m², at a dosage ranging from about 175 mg once a day to about 300 mg twice a day if the subject is less than twelve years of age and has a BSA of > 0.88-1.25 m², at a dosage ranging from about 75 mg once a day to about 150 mg t.i.d. if the subject is less than twelve years of age and has a BSA of > 0.73-0.88 m², at a dosage ranging from about 75 mg once a day to about 150 mg twice a day if the subject is less than twelve years of age and has a BSA of > 0.47-0.73 m², or at a dosage ranging from about 75 mg to about 150 mg once a day if the subject is less than twelve years of age and has a BSA of > 0.47 m².

### Definitions

As used herein, the term "treat" may be used to describe prophylaxis, amelioration, prevention or cure of a lysosomal storage disorder and disorders characterized by lysosomal dysfunction and/or one or more of its associated symptoms. For instance, treatment of an existing lysosomal storage disorder and disorders characterized by lysosomal dysfunction may reduce, ameliorate or altogether eliminate the disorder, or prevent it from worsening. Prophylactic treatment may reduce the risk of developing a disorder and/or lessen its severity if the disorder later develops.

A subject may be a rodent, a human, a livestock animal, a companion animal, or a zoological animal. In one embodiment, a subject may be a rodent, e.g., a mouse, a rat, a guinea pig, etc. In another embodiment, a subject may be a livestock animal. Non-limiting examples of suitable livestock animals may include pigs, cows, horses, goats, sheep, llamas and alpacas. In still another embodiment, a subject may be a companion animal. Non-limiting examples of companion animals may include pets such as dogs, cats, rabbits, and birds. In yet another embodiment, a subject may be a zoological animal. As used herein, a "zoological animal" refers to an animal that may be found in a zoo. Such animals may include non-human primates, large cats, wolves, and bears. In some preferred embodiments, a subject is a mouse. In other preferred embodiments, a subject is a human.

For the purpose of the invention, the term "pharmaceutically acceptable" is intended to mean what is useful to the preparation of a pharmaceutical composition, and what is generally safe and non-toxic, for a pharmaceutical use.

A "pharmaceutically acceptable carrier" or "excipient" or "solution" refers to a non-toxic solid, semi-solid or liquid filler, diluent, encapsulating material or formulation auxiliary of any type. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions of the invention contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The term "pharmaceutically acceptable salt" is intended to mean, in the framework of the present invention, a salt of a compound which is pharmaceutically acceptable, as defined above, and which possesses the pharmacological activity of the corresponding compound. The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

Within the meaning of this invention, "stereoisomers" is intended to designate diastereoisomers or enantiomers. These are therefore optical isomers. Stereoisomers which are not mirror images of one another are thus designated as "diastereoisomers," and stereoisomers which are non-superimposable mirror images are designated as "enantiomers".

Within the meaning of this invention, "conformers" is intended to designate a form of stereoisomers in which the isomers can be interconverted just by rotations about formally single bonds.

### FIGURE LEGENDS

**Figure 1** shows the effect of orally administered Trehalose, Miglustat, alone, and a combination of Trehalose and Miglustat, on neuronal cell death in the VPM / VPL area of CLN3 mice. The graph indicates the mean number of apoptotic cells / section detected by microscopy analysis using an antibody against cleaved caspase 3 (CASP3). Four male mice / group were analyzed. Data are presented as mean ± standard deviation. H= 7.2g high dose of Miglustat / kg chow; KO = knock-out; L= low dose of 1,2g Miglustat / kg chow; VPL = ventral posterolateral nucleus; VPM = ventral posteromedial nucleus.

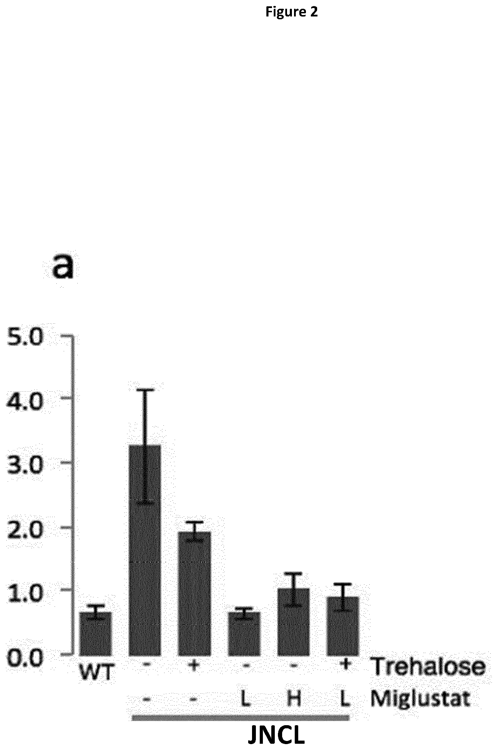

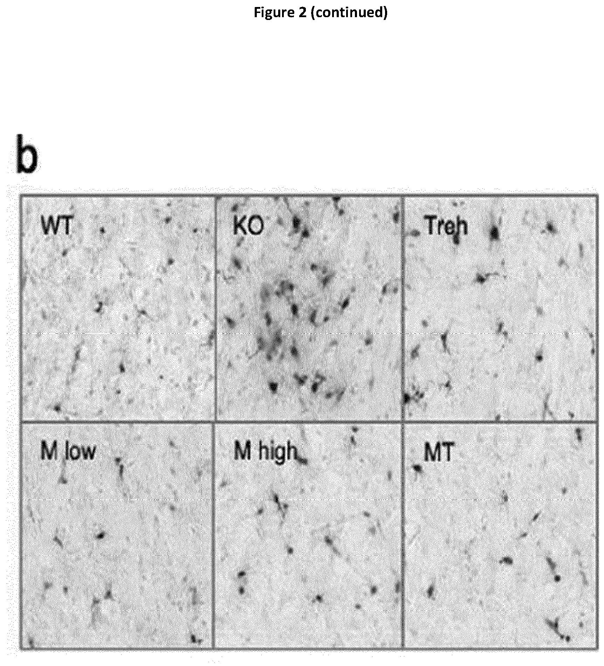

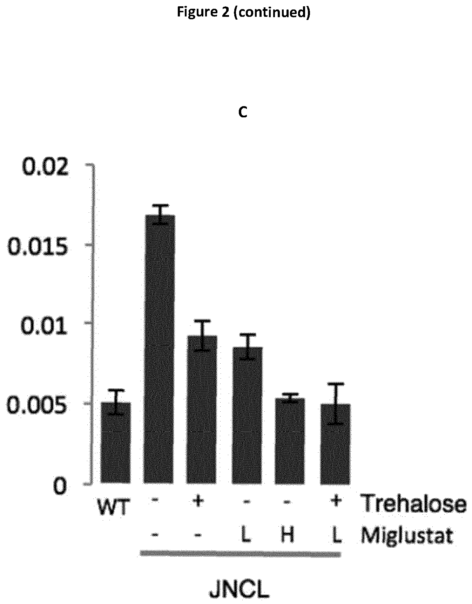

**Figure 2** shows the effect of Trehalose, Miglustat alone, and a combination of Trehalose and Miglustat on the microglial activation (CD68+ reactivity) within the thalamic ventral posterior medial and lateral nuclei (VPM/VPL) in Batten mice at 12 months of age. The graph a indicates the area / section positive to CD68 straining as detected by microscopy analysis. **a-b:** in the VPM/VPL region of the brain; c: in one region of the cerebellum of the mice. Four male mice / group were analyzed. Data are presented as mean ± standard deviation. H= 7.2g high dose of Miglustat / kg chow; L= low dose of 1,2g Miglustat / kg chow.

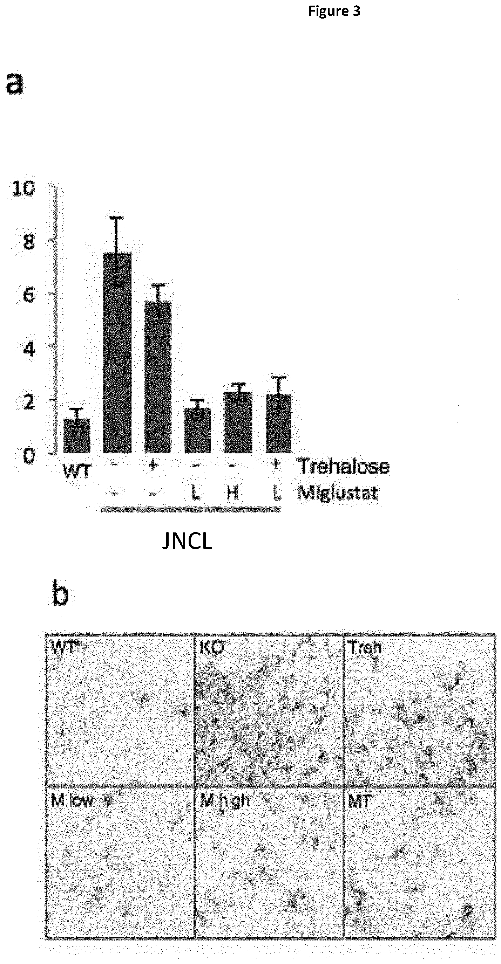

**Figure 3** shows the astroglial activation (GFAP staining) in the VPM/VPL region of untreated *Cln3*^{*Δex7*-8} mice at 12 months of age, which is partially prevented by Trehalose administration and largely suppressed by Miglustat alone and Miglustat/Trehalose administration. The graph **a** indicates the area / section positive to GFAP straining as detected by microscopy analysis. Representative microphotographs of GFAP straining in the analysed group are also presented in **b.** Four male mice / group were analyzed. Data are presented as mean ± standard deviation. H= 7.2g high dose of Miglustat / kg chow; L= low dose of 1,2g Miglustat / kg chow.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### 1. MATERIAL AND METHODS

### Mouse colony and treatments.

*Cln3*^{*Δex7*-8} mice were obtained from the Jackson Laboratory. Control (C57BL/6J) and *Cln3*^{*Δex7*-8} mice were housed 3-4 per cage in a room with a 12-h light/12-h dark cycle. Food and water were provided ad libitum. All mice used in this study were analyzed at 8 and 12 months of age and were littermates produced by crossing heterozygous *Cln3*^{*Δex7*-8} mice. Only males were used for this analysis. Investigators were blinded when analyzing the data, and no randomization was necessary.

For oral administration, Trehalose was dissolved in drinking water to a final concentration of 2% and changed twice a week. Trehalose-containing water was given to *Cln3*^{*Δex7*-8} mice and WT mice by spontaneous oral administration starting at 21 days of age and continuing until the day the mice were sacrificed for neuropathology studies.

Miglustat was administered in mixture with the food pellet starting at 21 days of age and continuing until the day the mice were sacrificed for neuropathology studies. Miglustat food was prepared by TestDiet using the 5V5R rodent diet (LabDiet). Two concentrations were used: 1.2 g/Kg chow (indicated as "low concentration") and 7.2 g/Kg chow (indicated as "high concentration") and two separate groups of *Cln3*^{*Δex7*-8} mice were fed with either diet. One additional group of *Cln3*^{*Δex7*-8} mice was fed with a combination of the low-concentration Miglustat food and 2% Trehalose water.

### Immunohistochemistry.

Twelve-month-old homozygous *Cln3*^{*Δex07*-8} mice and age-matched controls (WT mice) were anaesthetized with isoflurane and transcardially perfused with PBS followed by 4% buffered paraformaldehyde in 0.1 M sodium phosphate buffer, pH 7.4. Brains were subsequently removed and postfixed overnight. Before sectioning, the brains were cryoprotected in a solution containing 30% sucrose in Tris-buffered saline (TBS: 50 mM Tris, pH 7.6). Consecutive 40 µm floating coronal sections were collected in 96-well plates. Series of sections were then stained with primary antisera against CD68 (AbD Serotec, Cat. No. MCA1957), GFAP (DAKO, Cat. No. Z0334), Subunit C of mitochondrial ATP synthase (SCMAS; Abcam, Cat. No. ab181243), or cleaved caspase 3 (Asp175) (Cell Signaling, Cat. No. 9661S) followed by either rabbit anti-rat (VectorLab) or swine anti-rabbit (DAKO) secondary antibodies, and immunoreactivity detected with Vectastain ABC (avidin-biotin) kit (Vector) and diaminobenzidine as a chromogen.

### Quantitative microscopy analyses.

Non-overlapping images were captured, on three consecutive sections, through each region of interest. All RGB images were captured via a live video camera (JVC, 3CCD, KY-F55B), mounted onto a Zeiss Axioplan microscope using a x 40 objective and saved as JPEGs. All parameters including lamp intensity, video camera set-up and calibration were maintained constant throughout image capturing. For quantification of storage and inflammation, images were analyzed using ImageJ analysis software (NIH), using an appropriate threshold that selected the foreground immunoreactivity above background. This threshold was then applied as a constant to all subsequent images analyzed per batch of animals and reagent used to determine the specific area of immunoreactivity for each antigen in each region. Data were plotted graphically as the mean percentage area of immunoreactivity per field ± s.e.m. for each region. For quantification of cell death, images were analyzed manually and scored for the presence of apoptotic nuclei. Data were plotted graphically as the mean number of apoptotic cells per field ± s.e.m. for each region. All analyses were performed blind to genotype and treatment.

### Bioanalytical Methods for Analysis of In-Vivo Study Samples

LC-MS/MS bioanalytical methodologies were developed for the analysis of Trehalose, Miglustat and glucose in mouse whole blood and brain.

Blood samples were prepared as follows; 20 µL of whole blood was transferred to a 96 wellplate and diluted 1:1 with sterile distilled water. Samples were protein precipitated by addition of 160 µL of 2.5 % of trichloroacetic acid (TCA). At the time of analysis, 1 volume of acetonitrile containing an internal standard was added in order to match the initial chromatography conditions, samples were centrifuged at 4350 rpm for 10 min at ambient temperature, and the resultant supernatant transferred for analysis on the LC-MS/MS.

Tissue samples were homogenized in 8 volumes of sterile distilled water and 1 volume of TCA (20% in water) to generate a final concentration of 2% TCA, homogenates were centrifuged at 13000 rpm for 15 min at 4°C and the supernatant transferred to a fresh plate. At the time of analysis, 1 volume of acetonitrile containing an internal standard was added in order to match the initial chromatography conditions, samples were centrifuged at 4350 rpm for 10 min at ambient temperature, and the resultant supernatant transferred for analysis on the LC-MS/MS.

### II. RESULTS

### Example 1: Effect of orally administered Trehalose, Miglustat, and a combination of Trehalose and Miglustat on cell death in Batten mice.

Cysteinyl aspartate-specific protease 3 (CASP3), which is activated in the initiation and progression of programmed cell death, was used as an indicator of cell death in the presence of Trehalose + and - Miglustat. Trehalose and Miglustat were administered orally. Neuronal cell death was measured by the density [number of cells/area] of caspase-3 [CAS-3]-positive cells.

When treated with Trehalose + and - high and low doses of Miglustat (200mg/kg or 600mg/kg) and Miglustat alone, similar results were seen in the ventral posterior medial and the ventral posterolateral thalamic nuclei of WT mice and Trehalose-treated, Miglustat-treated Batten mice, and Batten mice treated with Trehalose plus Miglustat. The results shown in **Figure 1** show that the untreated *Cln3*^{*Δex7*-8} mice have increased cell death at 12 months of age, which is partially prevented by Trehalose administration and largely suppressed by Miglustat alone and Miglustat/Trehalose oral administration.

This study demonstrated that Miglustat alone, as well as the combination of Miglustat and Trehalose, are capable of inhibiting neuronal cell death and reducing neuroinflammation in a mouse model of CLN3 disease. Cleaved CAS-3 analysis in the VPM/VPL region demonstrate that untreated Cln3_{Δex7-8} mice experience increased percentages of cell death at 12 months of age over wild type animals. Disease-specific cell death is partially reduced by access to 2% Trehalose in drinking water and prevented by the administration of Trehalose and Miglustat.

### Example 2: Effect of Trehalose, Miglustat alone, and a combination of Trehalose and Miglustat on neuro-inflammation and cellular waste accumulation.

Chronic neuroinflammation is observed in both patients with CLN3 disease and nonclinical animal models. Data from animal models of CLN3 disease demonstrate early signs of glial activation that precedes neuronal loss (Pontikis et al., 2004). Inhibition of neuroinflammation can significantly attenuate axonal damage, neuron loss, retinal thinning, and brain atrophy in the CLN3 disease animals, suggesting that neuroinflammation may provide important extrinsic signals that influence neuronal function and survival during the disease (Groh et al., 2017).

One potential mechanism for Trehalose and Miglustat in the treatment of CLN3 disease is the reduction of neuroinflammation (microglial activation and astrogliosis), and subsequent amelioration of cell apoptosis within various areas of the CNS. The present study investigated the role of these drugs in reducing neuroinflammation in a mouse model of CLN3 disease.

Neuroinflammation (measured by the number of GFAP-positive astrocyte cells in the neuronal system), and microglia activation into the nervous system (measured by an increase in CD68 staining) were evaluated in wild-type and CLN3-deficient mice.

To investigate whether Trehalose + or - Miglustat inhibits neuroimmune responses in juvenile Batten disease, microglial activation (CD68+ reactivity) was examined in the ventral posterior medial and the ventral posterolateral thalamic nuclei, the cerebellum in Batten mice (**figure 2c**). The results show that the untreated *Cln3*^{*Δex7*-8} mice have increased neuroinflammation in the VPM/VPL region (**figure 2** **a** and **b**) and in the cerebellum (**figure 2c**) at 12 months of age. Trehalose administration partially reduces CD68 staining in the VPM/VPL and in some areas of the cerebellum. Administration of Miglustat (low or high concentration) largely reduces CD68 staining in the VPM/VPL (**figure 2** **a** and **b**) and partially or largely reduces CD68 staining in various regions of the cerebellum (**figure 2c**). Simultaneous administration of Trehalose and Miglustat largely reduces CD68 staining in the VPM/VPL (**figure 2** **a** and **b**) and in some regions of the cerebellum (**figure 2c**).

In addition to increased microglial activation, previous reports describe clusters of GFAP+ astrocytes with extended reactive processes across the cerebellum with disordered Purkinje cell processes and the loss of Purkinje cells themselves. The analysis of astroglial activation (GFAP staining) shows that the untreated *Cln3*^{*Δex7*-8} mice have increased astrocytosis in the VPM/VPL region at 12 months of age, which is partially prevented by Trehalose administration and largely suppressed by Miglustat alone and Miglustat/Trehalose administration (**figure 3**).

Additionally, Miglustat is a known inhibitor of glycosphingolipid synthesis in the brain and is able to decrease the erroneous accumulation of harmful quantities of GM2 gangliosides ie Gaucher disease. Patients with juvenile Batten disease also exhibit accumulation of GM2 gangliosides to a lesser extent. Changes in the accumulation of harmful quantities of GM2 gangliosides in the Cerebellum were assessed by immunodetection of subunit C of ATPase, a major component of juvenile Batten inclusion bodies in the Cerebellum. The analysis of aberrant lysosomal storage (SCMAS staining) shows that the untreated *Cln3*^{*Δex7*-8} mice have increased storage in the cerebellum (**data not shown**) at 12 months of age. Administration of Trehalose partially reduces the storage, whereas Miglustat has no consistent effects on storage levels. Simultaneous administration of Trehalose and Miglustat consistently obtains the best clearance effects. Previously thought of as minimal and therefore not harmful, recent evidence suggests that systemic accumulation of harmful gangliosides may be reduced by daily doses of Miglustat.

### Example 3: Pharmacokinetic and pharmacodynamic results for administration of oral and intravenous Trehalose.

Due to the poor PO bioavailability of Trehalose, it was proposed to use IV dosing of Trehalose in the clinic alongside PO doses of Miglustat. A study was conducted in mouse with PO and IV doses of Trehalose conducted with and without PO doses of Miglustat.

The purpose of this example was to compare the blood PK of unlabelled high doses of Trehalose after IV and PO administration, with and without Miglustat PO dosing. For the IV comparison, Trehalose was dosed as an IV bolus dose 15 min after PO dosing of Miglustat. For the PO comparison Miglustat was dosed simultaneously with Trehalose. Fasted male C57BL6/N mice were dosed as detailed in Table III:

| **Group** | **Route** | **Dose** | **Formulation** | **n** |
|---|---|---|---|---|
| **1** | **IV** | **1 g/kg trehalose** | **Sterile distilled water 200 mg/mL trehalose** | **4** |
| **2** | **PO/IV** | **150 mg/kg miglustat PO & 1 g/kg trehalose IV** | **Sterile distilled water 15 mg/mL miglustat (PO) & 200 mg/mL trehalose (IV)** | **4** |
| **3** | **PO** | **10 g/kg trehalose** | **Sterile distilled water 1g/mL trehalose** | **4** |
| **4** | **PO** | **10 g/kg trehalose & 150 mg/kg miglustat** | **Sterile distilled water 1g/mL trehalose & 15 mg/mL miglustat** | **4** |

Blood samples were collected at pre-dose, 10, 20, 30, 60, 120 and 180 min post-dose for group 1 and 2 and at pre-dose, 15, 30, 45, 60, 120 and 180 min post-dose for group 3 and 4.

It is important to notice that the Trehalose dose for oral administration is ten times higher than the dose for IV injection. This is because low doses of Trehalose such as 1g/kg cannot be detected after oral administration.

Blood concentrations of Trehalose were analysed with LC-MS/MS and PK parameters are summarized in Table IV:

| **Dose (g/kg) & Route** | **Cₘₐₓ (µM)** | **Tₘₐₓ (min)** | **AUC_{0-inf}(µM.min)** | **Trehalose fold-change with miglustat dosing** |
|---|---|---|---|---|
| **1 IV** | 3689.2 ± 152.7 | 20 | 117389.3 ± 20735.8 | - |
| **1 IV + miglustat** | **5494.3** ± 873.3 | 20 | 179501.0 ± 17152.8 | 1.5 |
| **10 PO** | 148.4 ± 73.2 | 15 | 11170.5 ± 3541.6 | - |
| **10 PO** + **miglustat** | 188.6 ± 43.9 | 15 | 18394.3 ± 4560.5 | 1.6 |

The Cₘₐₓ results clearly show that oral administration of Trehalose is much less efficient than IV administration, as the level of Trehalose in blood is 25 folds less even if 10 times more of Trehalose has been administered.

The results moreover show that the combined administration of PO Miglustat with Trehalose *via* either IV or PO administration significantly increased the measured AUC of Trehalose (>1.5 fold). Yet, the administration of Miglustat does not enhance the level of PO Trehalose to a sufficient level, even if 10 times more Trehalose is administered: the Cₘₐₓ results clearly show that combination of oral administration of Trehalose and Miglustat is still much less efficient than when Trehalose is administered by IV (with or without Miglustat), as the level of Trehalose in blood is 29 folds less even if 10 times more of Trehalose has been administered.
Tissue concentrations of Trehalose were analysed with LC-MS/MS and PK parameters are summarized in Table V :

| **Dose (g/kg) & Route** | **Concentration (nmol/g) Liver** | **Concentration (nmol/g) Brain R** | **Concentration (nmol/g) Brain L** |
|---|---|---|---|
| **1 IV** | 167.9 ± 24.b | 7.3 ± 0.9 | 6.9 ± 19 |
| **1 IV + miglustat** | 278.6 ± 96.6 | 10.5 ± 1.7 | 10.8 ± 2.0 |
| **10 PO** | 191.0 ± 21.2 | 2.6 ± 1.2 | 2.5 ± 1.0 |
| **10 PO +** miglustat | 142.7 ± 30.2 | 3.3 ± 1.2 | 3.3 ± 1.2 |

Tissue levels of Trehalose were above the limit of detection in all analysed samples. To assess variability in sample preparation right and left brain hemispheres were analysed separately demonstrating high reproducibility and low variability.

The results clearly show that administration of Trehalose *via* the IV route led to higher levels in the brain compared to PO dosing (again, although 10 times less Trehalose was administered). Also, the results show that, in combination with oral Miglustat, the brain exposure of Trehalose is significantly increased when measured at 180 min post-dose (from about 7 to about 10 nmol/g). This was surprising that the greatest brain exposures were achieved through IV compared to oral dosing (about 7 nmol/g by IV versus 2.5 nmol/g by PO). This demonstrates that intravenous administration of Trehalose could lead to a significant increase in brain exposure, despite what was described in the art.

It was also demonstrated that oral administration of Miglustat (150 mg/kg) increased Trehalose exposure in brain tissue after oral and IV administration of Trehalose and that IV administration of Trehalose continues to provide the greatest exposures in brain tissue at the same doses. It has been found that, surprisingly, combining Miglustat with oral Trehalose does not increase plasma or brain tissue exposure to the same extent as for the combination or miglustat with IV trehalose.

Thus, only IV administration of Trehalose combined with oral application of Miglustat can significantly increase Trehalose brain tissue exposure in WT C57BL/6 mice. Without being bound to this theory, this effect is possible due to the fact that Miglustat can not only prevent Trehalose from being hydrolysed by the Trehalase enzyme present in the plasma, but it can also stabilize Trehalose in the brain. Miglustat crosses the blood-brain barrier and has been shown to be distributed to the brain.

## Claims

1. A combination product comprising Trehalose and Miglustat, or any pharmaceutically acceptable salt thereof, for simultaneous, separated, or staggered use for treating a lysosomal storage disorder or a disorder **characterized by** lysosomal dysfunction in a subject in need thereof, wherein Trehalose is administered parenterally and Miglustat is administered orally.

2. The combination product of claim 1, wherein said disorders are chosen in the group consisting of : Juvenile Neuronal Ceroid Lipofuscinosis (JNCL, juvenile Batten or CLN3 disease), Aspartylglucosaminuria, Cystinosis, Fabry Disease, San Filippo disease, Gaucher Disease Types I, II, and III, Glycogen Storage Disease II (Pompe Disease), GM2-Gangliosidosis Type I (Tay Sachs Disease), GM2-Gangliosidosis Type II (Sandhoff Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN11 disease, CLN12 disease, CLN13 disease, and CLN14 disease.

3. The combination product of claim 2, wherein said disorders are chosen in the group consisting of Neuronal Ceroid Lipofuscinosis, such as CLN1 disease, CLN2 disease, CLN3 disease, CLN4 disease, CLN5 disease, CLN6 disease, CLN7 disease, CLN8 disease, CLN10 disease, CLN12 disease, CLN13 disease, and CLN14 disease, and is preferably Juvenile Neuronal Ceroid Lipofuscinosis (JNCL, juvenile Batten or CLN3 disease).

4. The combination product of any one of claim 1 to 3, wherein Trehalose is the single active principle in the composition for parenteral administration, which optionally further comprises at least one pharmaceutically acceptable additive, carrier, excipient or diluent.

5. The combination product of any one of claim 1 to 4, wherein Miglustat is the single active principle in the composition for oral administration, which optionally further comprises at least one pharmaceutically acceptable additive, carrier, excipient or diluent.

6. The combination product of any one of claim 1 to 5, wherein Trehalose is intravenously administered.

7. The combination product of claim 6, wherein Trehalose is intravenously administered at a dosage comprised between 0,25 - 0,75 g/kg, once weekly.

8. The combination product of claim 7, wherein Trehalose is administered by an intravenous infusion extending for about 25 minutes to about three hours, preferably for about 50 to about 70 minutes.

9. The combination product of any one of claim 1 to 8, wherein Miglustat is orally administered in a capsule.

10. The combination product of claim 9, wherein Miglustat is administered at a dosage ranging from about 300 to 600 mg per day.

11. The combination product of claim 9, wherein Miglustat is administered three to six times per day, preferably with three to six capsules containing 100 mg of Miglustat.

12. The combination product of any one of claims 1 to 11, wherein:
- Trehalose is administered intravenously once a week at a dosage comprised between 0,25 - 0,75 g/kg, preferably for about 50 to about 70 minutes,
- Miglustat is administered orally three times to six times per day, in capsules containing 100 mg of Miglustat.

13. The combination product of any one of claims 1 to 12, wherein:
- Trehalose is administered intravenously once a week at a dosage comprised between 0,25 - 0,75 g/kg, preferably for about 50 to about 70 minutes,
- Miglustat is administered orally three times per day, in capsules containing 100mg of Miglustat.
